# EUROPEAN PATENT SPECIFICATION

(11) **EP 2 364 713 B1**
(45) Date of publication and mention of the grant of the patent: **30.04.2014**
(21) Application number: 10153915.3
(22) Date of filing: 18.02.2010
(51) Int. Cl.: A61K 9/00, A61K 9/06, A61K 36/185, A61K 36/35, A61K 36/38, A61K 36/63

(54) **Dermatological composition for use in the treatment of skin burns, skin diseases and infected wounds**
Dermatologische Zusammensetzung zur Verwendung bei der Behandlung von Hautverbrennungen, Hauterkrankungen und infizierten Wunden
Composition dermatologique à utiliser dans le traitement des brûlures de la peau, maladies de la peau et plaies infectées

(30) Priority: 05.11.2009 TR 200908386
(43) Date of publication of application: 14.09.2011
(73) Proprietor: MCI Ilaç ve Kozmetik Ürünleri Imalat Ticcaret Limited Sirketi, 16110 Bursa (TR)
(72) Inventor: Aycan, Ilkay, 16110, Bursa (TR)
(74) Representative: Iskender, Ibrahim

(56) References cited:
- WO-A2-2006/013607
- ES-A1- 2 052 443
- US-A1- 2007 122 492
- US-A1- 2008 199 489
- HALCON L ET AL: "Staphylococcus aureus and wounds: A review of tea tree oil as a promising antimicrobial", AMERICAN JOURNAL OF INFECTION CONTROL, C.V. MOSBY CO., ST. LOUIS, MO, US, vol. 32, no. 7, 1 November 2004 (2004-11-01), pages 402-408, XP004620846, ISSN: 0196-6553, DOI: DOI:10.1016/J.AJIC.2003.12.008
- KIRAN K; ASAD M: "Wound healing activity of Sesamum indicum L seed and oil in rats", INDIAN JOURNAL OF EXPERIMENTAL BIOLOGY, vol. 46, no. 11, November 2008 (2008-11), pages 777-782, XP002655058, ISSN: 0019-5189

## Description

### Technical Field

The invention relates to a multipurpose dermatological composition used in the treatment of skin burns, skin diseases and infected wounds, which enables the wounds and burns to heal without forming the cicatrix tissue (without scabbing) and without leaving a scar, and comprises the elderberry tree bark extract (Sambucus nigra L.), hypericum perforatum oil, simmondsia californica (jojoba) oil, olive oil, sesamum indicum (sesame) oil, melaleuca alternifolia oil, beeswax (cera alba), lavandula officinalis oil and gum mastic (pistascia lentiscus).

### Prior Art

The burns, infected wounds, chaps, cuts, non-allergic skin lesions, eczema and fungus infections, which occur in the skin and usually result from the external factors, interrupt the normal functions of the tissues, thereby leading to their destruction or injury.

The literature includes many studies developed for use in the treatment of the wounds and the burns. As an example, in the patent application number W00152872, the compound that is related with pharmaceutics and consists of ingredients such as olive oil, distilled water, animal suet, bee wax, sambucus nigra, paterium sanguisorba, veronica officinalis and polypodium vulgare is used for treatment of topical damages on the skin, especially on burn injuries.

The ingredients of the mentioned composition are freshly picked, chopped and heated for 1 to 8 hours. Simultaneously extraction process is performed by constant mixing under aseptic conditions. However, after the usage of this herbal composition, the deformation on the cicatrix tissue is not completely healed, become permanent and leaves an existing scar on life time. The mentioned compositions are also not effective on high degree burns and are not capable enough for relieving pain and burn sensation.

For this reason Dr. llkay Aycan developed patent application, TR 2008 00328 which is a dermatologic composition for treatment of above conditions and infected wounds and includes, sambucus nigra bark extract, animal suet, herbal oil, bee wax and gum mastic. However, the fact that one of the ingredients, animal suet can not get standardized, it is prohibited to be used in herbal compound formulas. Therefore alternative oils with standard forms that can be found easily and can compensate the animal suet should be used in the composition..

As a result, requirement to an improvement which eliminates the disadvantages of the existing technique by healing the burns, skin dermatitis, and infected wounds without having a cicatrix tissue and without leaving a scar in a short period of time became necessary due to the insufficiency of the results of existing techniques.

### Object of the Invention

Based on the state of the art, the object of the invention is to develop a multipurpose dermatological composition, which is used in the treatment of the skin burns, skin diseases and infected wounds, and is able to provide 100% healing without causing any side effect on the skin where it is applied.

Another object of the invention is to develop a multipurpose composition, which may be stored at room temperature for years without loosing its features, but may melt at the body temperature.

Another object of the invention is to develop a multipurpose composition, which may be easily applied on the skin owing to its oily structure and may provide convenience in changing the wound dressing as it does not adhere onto the skin.

Another object of the invention is to develop a multipurpose composition, which reduces the risk of death even in the burn cases with the danger of mortality, where 20% and more of the body surface are burned.

Another object of the invention is to develop a multipurpose composition, which enables the skin burns, skin diseases and infected wounds to heal without forming a cicatrix tissue (without scabbing) and without leaving a scar, as opposed to the existing treatment methods.

Still another object of the invention is to develop a multipurpose composition, which speeds up the healing process for the skin burns, skin diseases and infected wounds 3-4 times as compared to normal medical treatments, and completely heals the simple burns and cuts within one to two days.

Still another object of the invention is to develop a multipurpose composition, which ceases the sense of pain and burning within couple hours, in the incidents of burn.

Still another object of the invention is to develop a multipurpose composition, which has practical manufacturing steps and a low manufacturing cost.

In order to achieve said objects, the invention is a dermatological composition used in the treatment of the skin burns, skin diseases and infected wounds, which enables the wounds and burns to heal without forming the cicatrix tissue (without scabbing) and without leaving a trace, comprising the elderberry bark extract and olive oil and it is characterized in that it comprises hypericum perforatum oil, melaleuca alternifolia oil and sesamum indicum (sesame) oil.

According to a preferred embodiment of the invention, said composition also comprises the simmondsia californica (jojoba) oil and/or cera alba (beeswax) as thickener.

According to a preferred embodiment of the invention, said jojoba oil is the pure, natural jojoba oil.

According to a preferred embodiment of the invention, said composition also comprises the odorizing gum mastic (pistascia lentiscus) and lavandula officinalis oil.

The dermatological composition comprising the elderberry bark extract, olive oil, hypericum perforatum oil, melaleuca alternifolia oil and sesamum indicum oil developed to achieve said objects is used in the production of "Derion" , "Stigma" , "Recontour" named cosmetic creams, sprays and ointments for the treatment of the skin burns, skin diseases and infected wounds.

### Detailed Description of Invention

The invention relates to a multipurpose dermatological composition used in the treatment of the skin burns, skin diseases and infected wounds, which enables the wounds and burns to heal without forming the cicatrix tissue (without scabbing) and without leaving a scar.

Said composition comprises the elderberry bark extract, hypericum perforatum oil, melaleuca alternifolia oil, sesamum indicum oil, olive oil, simmondsia californica oil (jojoba oil), cera alba (beeswax), lavandula officinalis oil and pistascia lentiscus (gum mastic).

The jojoba oil and beeswax are used according to the invention as thickener.

Olive oil is used as vegetable oil, which nourishes the skin and also moisturizes the skin to prevent the stretching and drying. The olive oil must be pure. The thinned refined olive oil is not used. Gum mastic and lavandula officinalis oil have the odorizing characteristic.

In the Table 1 below, the components making up the composition according to the invention and their amounts are provided.

**Table1. Components Making Up the Composition According to the Invention and Their Amounts**

| Ingredient | Preferred amount | Usable amount |
|---|---|---|
| Elderberry bark extract | 20 gr | 10-100 |
| Jojoba oil | 250 gr | 150-500 |
| Hypericum perforatum oil | 100 gr | 50-250 |
| Melaleuca alternifolia oil | 100 gr | 50-250 |
| Sesamum indicum oil | 100 gr | 50-250 |
| Olive oil | 200 ml | 100-300 |
| Beeswax | 100 gr | 50-100 |
| Gum Mastic | 2 gr | 1 -10 |
| Lavandula ofificinalis oil | 2 gr | 1 -10 |

The Latin name of the main ingredient elderberry tree is sambucus nigra. It is also called as black elderberry in some locations of Anatolia. The flowers blooming in May and June shall be picked up with their stems. Elderberries when consumed as tea, is effective for constipation, myx expectorant, urine expectorant and sudoriparous. Its sudariparous feature is beneficial in cold and influenza. Sambucus nigra flower tea is also recommended in hay fever, dermatitis of upper respiratory tract and sinusitis. Hearing disorders due to the flu symptoms can be treated by sambucus nigra flower tea. Due to its effect on immune system strengthening, it is taken to prevent cold and influenza. It is proven in literature that lectin in sambucus nigra is effective on cell construction in various ways and it is especially effective on RNA synthesis. Mechanism of this effect is related with DNA repair in the cells. In the laboratory tests, the inflammation controlling effect is also observed.

Hypericum perforatum is a natural antidepressant for minor and moderate level depressions. It is thought that, the active ingredient hypericin in the plant is effective for depression. Although it is not completely known how the effect mechanism of hypericum perforatum works, it is known that it treats by increasing the serotonin.

The flowers and the leaves of the plant have the active substance. The English name of the hypericum perforatum is St. John's Wort.

Hypericum perforatum is effective for viruses and has relieving influence on discomfort and pain before menstrual periods. Another areas that hypericum perforatum is good for are burns and wounds. It is recommended in menopause syndrome. It is also topically used for muscle pain and soft tissue rheumatism. Hypericum perforatum oil can be topically applied on various cases such as; open wounds, new wounds, dermatitis, swelled glands, sun burns, skin deformations on face, cold sores, varicose veins, hemorrhoids, back pains, lumbago, sciatica, joint infections, rheumatics and paralysis. The flowers of the hypericum perforatum are put in flaxseed oil to be effectively used on burns. It can also be used on sunburns.

Melaleuca alternifolia oil is a very effective topical antibacterial and fungal blocker. It is also used on warts.

Jojoba oil is obtained from jojoba plant which is the general name as well as the only name of spurge plant family. It is a product that is grown on semi dry soil. The jojoba plant exists in the deserts of United States of America and Mexico. The value of the jojoba plant comes from the jojoba oil which is produced from seeds by approximately 50%. Jojoba oil is used for lotions, shampoos and hair jells. Jojoba oil can also be used as lubricant and on various other applications. It can be manufactured as vegetable oil format or in a wax format by getting hydrogenised. Jojoba plant can reach up to 20 feet and grows in 10 years. It is raised in Arizona, California, Mexico, some Asian countries, Ghana and Australia. It is widely used in many of the products of cosmetic firms.

The chemical nature of the jojoba oil is very similar with the natural oil in human skin. That's why; jojoba oil is a very effective moisturizer. It does not leave oil remainders on the skin. Jojoba oil is very suitable for massage. Jojoba oil smoothes hair and prevents hair from braking.

Jojoba oil consist of oil acid and esters, it is rich in unsaturated oil acids. Because of its high viscosity nature it helps moisturizing the skin. It helps refreshing the irritated skin after shaving and balancing skin moisture level. It is a herbal supporting substance for hyperkeratos and eliminate wrinkles which are commonly observed on smokers. It is antiallergenic. It is recommended as main oil for preventing stretch marks after pregnancy. Jojoba oil smoothes the skin, and used to minimize wrinkles and lines. It moisturizes the dry skin and eliminates acnes.

Beeswax, another component of the invention, has the fatty acid and alcohol as the principle ingredients. It also includes propolis, color and a high amount of vitamin A. Vitamin A may be taken up by chewing the beeswax. The wax made of beeswax has been associated with high value since the very old times, because it does not irritate the eyes and it does not cause tickle and burning in the throat. It is also recommended to burn the candles made of fresh beeswax in the rooms of the patients with cough, pertussis, asthma, sinusitis, cold and hay fever, since it clears the respiratory organs and prevents the formation of sputum.

Sesame oil is obtained by squeezing the seeds in cold process. The content of the sesame oil is rich in E, A, and B vitamins, iron and calcium minerals, oleic, linoleic, stearic, palmitic, and miristic fatty acids and antioxidant containing compounds. With the ingredients of oleic acid (omega-9) and antioxidants, sesame oil helps to prevent skin aging and to provide long lasting moisture.

The sun protection factor of sesame oil is SPF 45 which is quite high. When mixed with cacao oil it can provide healthy and long lasting sun tanning and protects skin from the harmful effects of see and sun. The ingredients of sesame oil such as, iron, lecithin and vitamins help resolution of anemia problems. Calcium in the sesame oil helps overcoming the bone density loss as a result of aging. Topical usage of the sesame oil also helps preventing various skin problems and fungus infections. It also helps repairing the deformed nails due to the lack of calcium and vitamin.

During the production of the composition according to the invention, the branches of the elderberry are cut and their barks are peeled. The peeled barks are processed in extract manufacturing facility to get the sambucus nigra bark extract. The sambucus nigra extract is then mixed with hypericum perforatum oil, melaleuca alternifolia oil and sesamum indicum oil followed by the addition of olive oil. After the compound is homogenized, jojoba oil, beeswax, gum mastic and lavandula officinalis oil are added to get the cream viscosity. The obtained composition is than injected in tubes and becomes ready for use.

The composition can be directly applied on the wound. Until the wound or the burn heals, once or twice a day, the wound dressing is removed and is washed with water or preferably hydrogen peroxide, and the wound dressing process is repeated. In case said composition may not be directly drawn on the wound or the burn due to the pain and ache, it is drawn on the gauze bandage and the gauze is placed onto the wound or the burn. The treatment method is the same for all the wounds and burns. In addition to the skin burns and infected wounds, the composition according to the invention may also be used in the treatment of any non-allergic skin lesion, chaps in skin, heel and nipples, cuts, eczema and fungus infections, and even in the treatment of the skin diseases like onychomycosis, decubitus ulcers, skin ulceration associated with diabetes, furuncle and carbuncle, with long term use, and in healing the surgery marks.

The protection scope of this application is set forth in the section of claims and the scope may by no means be limited to the description above provided only for exemplary purposes. It is obvious that a person skilled in the art may provide the innovation put forward by the invention also by using the similar embodiments and/or apply this embodiment to other fields with similar purpose used in the relevant art. Consequently, such embodiments would obviously lack of novelty and particularly, inventive step criteria.

## Claims

1. Dermatological composition for use in the treatment of the skin burns, skin diseases and infected wounds, which enables the wounds and burns to heal without forming the cicatrix tissue (without scabbing) and without leaving a scar, comprising elderberry bark extract and olive oil, **characterized in that** it comprises the hypericum perforatum oil, melaleuca alternifolia oil and sesamum indicum oil.

2. Composition for use according to Claim 1 **characterized in that** it also comprises jojoba oil and/or beeswax as thickener.

3. Composition for use according to Claim 2 **characterized in that** said jojoba oil is pure and herbal jojoba oil.

4. Composition for use according to Claim 1 **characterized in that** it also comprises the odorizing gum mastic and/or lavandula officinalis oil.

5. Use of the dermatological composition comprising the elderberry bark extract, olive oil, hypericum perforatum oil, melaleuca alternifolia oil and sesamum indicum oil in the production of a medicament for the treatment of the skin burns, skin diseases and infected wounds.

6. Use according to Claim 5 **characterized in that** said composition comprises the jojoba oil and/or beeswax as thickener.

7. Use according to Claim 6 **characterized in that** said jojoba oil is pure and herbal jojoba oil.

8. Use according to Claim 5 **characterized in that** said composition also comprises the odorizing gum mastic and/or lavandula officinalis oil.

## Patentansprüche

1. Dermatologische Zusammensetzung zur Verwendung bei der Behandlung von Hautverbrennungen, Hautkrankheiten und infizierten Wunden, welche es den Wunden und Verbrennungen ermöglicht zu heilen, ohne das Zikatrixgewebe zu bilden (ohne Abschuppung) und ohne eine Narbe zu hinterlassen, umfassend Holunderrindenextrakt und Olivenöl, **dadurch gekennzeichnet, dass** sie das Hypericum-perforatum-Öl, Melaleuca-alternifolia-Öl und Sesamum-indicum-Öl umfasst.

2. Zusammensetzung zur Verwendung nach Anspruch 1, **dadurch gekennzeichnet, dass** sie außerdem Jojobaöl und/oder Bienenwachs als Verdickungsmittel umfasst.

3. Zusammensetzung zur Verwendung nach Anspruch 2, **dadurch gekennzeichnet, dass** das Jojobaöl reines und pflanzliches Jojobaöl ist.

4. Zusammensetzung zur Verwendung nach Anspruch 1, **dadurch gekennzeichnet, dass** sie außerdem das odorierende Mastix und/oder Lavandula-officinalis-Öl umfasst.

5. Verwendung der dermatologischen Zusammensetzung, umfassend den Holunderrindenextrakt, Olivenöl, Hypericum-perforatum-Öl, Melaleuca-alternifolia-Öl und Sesamum-indicum-Öl, bei der Herstellung eines Medikaments zur Behandlung der Hautverbrennungen, Hautkrankheiten und infizierten Wunden.

6. Verwendung nach Anspruch 5, **dadurch gekennzeichnet, dass** die Zusammensetzung das Jojobaöl und/oder Bienenwachs als Verdickungsmittel umfasst.

7. Verwendung nach Anspruch 6, **dadurch gekennzeichnet, dass** das Jojobaöl reines und pflanzliches Jojobaöl ist.

8. Verwendung nach Anspruch 5, **dadurch gekennzeichnet, dass** die Zusammensetzung außerdem das odorierende Mastix und/oder Lavandula-officinalis-Öl umfasst.

## Revendications

1. Composition dermatologique à utiliser dans le traitement de brûlures de la peau, de maladies de la peau et de plaies infectées, qui permet aux plaies et aux brûlures de cicatriser sans former de tissu cicatriciel (sans formation de croûte) et sans laisser une cicatrice, comprenant de l'extrait d'écorce de sureau et de l'huile d'olive, **caractérisé en ce qu'**elle comprend de l'huile d'hypericum perforatum, de l'huile de melaleuca alternifolia et de l'huile de sesamum indicum.

2. Composition à utiliser selon la revendication 1, **caractérisée en ce qu'**elle comprend également de l'huile de jojoba et/ou de la cire d'abeilles comme agent épaississant.

3. Composition à utiliser selon la revendication 2, **caractérisée en ce que** ladite huile de jojoba est de l'huile de jojoba pure et végétale.

4. Composition à utiliser selon la revendication 1, **caractérisée en ce qu'**elle comprend également de la gomme mastic odorante et/ou de l'huile de lavandula officinalis.

5. Utilisation de la composition dermatologique comprenant l'extrait d'écorce de sureau, l'huile d'olive, l'huile d'hypericum perforatum, l'huile de melaleuca alternifolia et l'huile de sesamum indicum dans la production d'un médicament pour le traitement des brûlures de la peau, des maladies de la peau et des plaies infectées.

6. Utilisation selon la revendication 5, **caractérisée en ce que** ladite composition comprend l'huile de jojoba et/ou la cire d'abeilles comme agent épaississant.

7. Utilisation selon la revendication 6, **caractérisée en ce que** ladite huile de jojoba est de l'huile de jojoba pure et végétale.

8. Utilisation selon la revendication 5, **caractérisée en ce que** ladite composition comprend également la gomme mastic odorante et/ou l'huile lavandula officinalis.
